# EUROPÄISCHE PATENTSCHRIFT

(11) **EP 0 933 429 B1**
(45) Veröffentlichungstag und Bekanntmachung des Hinweises auf die Patenterteilung: **10.12.2003**
(21) Anmeldenummer: 99890004.7
(22) Anmeldetag: 15.01.1999
(51) Int. Cl.: C12N 15/86, C12N 7/01, A61K 48/00

(54) **Chimäres Poxvirus, das ein retrovirales Vektorgenom enthält**
Chimeric poxvirus comprising a retroviral vector genome
Poxvirus chimerique comprenant le génome d'un vecteur rétroviral

(30) Priorität: 16.01.1998 AT 5698
(43) Veröffentlichungstag der Anmeldung: 04.08.1999
(73) Patentinhaber: Baxter Aktiengesellschaft, 1221 Wien (AT)
(72) Erfinder: Falkner, Falko-Günter, Dr., 2304 Orth/Donau (AT); Holzer, Georg, Dr., 1180 Wien (AT); Dorner, Friedrich, Prof., 1230 Wien (AT)
(74) Vertreter: Alge, Daniel, Mag. Dr. rer.nat.

(56) Entgegenhaltungen:
- HIRSCH V. M. ET AL.: "Patterns of viral replication correlate with outcome in Simian Imunodeficiency Virus (SIV)-infected macaques: Effect of prior immunization with a trivalent SIV vaccine in modified Vaccinia virus Ankara." JOURNAL OF VIROLOGY, Bd. 70, Nr. 6, Juni 1996, Seiten 3741-3752, XP002102926
- NOGUIEZ-HELLIN P. ET AL.: "Plasmoviruses: Nonviral/viral vectors for gene therapy." PROC. NATL. ACAD. SCI. USA, Bd. 93, April 1996, Seiten 4175-4180, XP002102927
- HAFFAR O. ET AL.: "HUMAN IMMUNODEFICIENCY VIRUS-LIKE, NONREPLICATING, GAG-ENV PARTICLES ASSEMBLE IN A RECOMBINANT VACCINIA VIRUS EXPRESSION SYSTEM" JOURNAL OF VIROLOGY, Bd. 64, Nr. 6, 1. Juni 1990, Seiten 2653-2659, XP002027078
- KARACOSTAS V. ET AL.: "HUMAN IMMUNODEFICIENCY VIRUS-LIKE PARTICLES PRODUCED BY A VACCINIA VIRUS EXPRESSION VECTOR" PROCEEDINGS OF THE NATIONAL ACADEMY OF SCIENCES OF USA, Bd. 86, Nr. 22, 1. November 1989, XP000080977
- WAHLFORS J. J. ET AL.: "Semlike Forest virus-mediated production of retroviral vector RNA in retroviral packaging cells." HUMAN GENE THERAPY, Bd. 8, 20. November 1997, Seiten 2031-2041, XP002102928
- CHUAH M. K. L. ET AL.: "DEVELOPMENT AND ANALYSIS OF RETROVIRAL VECTORS EXPRESSING HUMAN FACTOR VIII AS A POTENTIAL GENE THERAPY FOR HEMOPHILIA A" HUMAN GENE THERAPY, Bd. 6, Nr. 11, 1. November 1995, Seiten 1363-1377, XP000570044
- BILBAO G. ET AL.: "Adenoviral/retroviral vector chimeras: a novel strategy to achieve high-efficiency stable transduction in vivo" FASEB JOURNAL, Bd. 11, 11. November 1997, Seiten 624-634, XP002091318
- HOLZER G. W. ET AL.: "Poxviral / retroviral chimeric vectors allow cytoplasmic production of transducing defective retroviral particles." VIROLOGY, Bd. 253, Nr. 1, 5. Januar 1999, Seiten 107-114, XP002102929

## Beschreibung

Die Erfindung betrifft ein chimäres Poxvirus enthaltend die Vektorkomponenten eines retroviralen (Defekt)-Genoms. Desweiteren betrifft die Erfindung ein chimäres Poxvirus enthaltend die Vektor- und Verpackungskomponenten zur Produktion von retroviralen Defektviren sowie die Verwendung des erfindungsgemäßen chimären Poxvirus für die Gen- oder Tumortherapie.

Ein zentrales Ziel in der Gentherapie ist die stabile genetische Modifikation von Zielzellen, wobei für gentherapeutische Anwendungen bislang vor allem virale Vektoren auf Basis von replikationsdefizienten Adenoviren und Retroviren konstruiert wurden.

Der in vivo-Gentransfer ist mit adenoviralen Vektoren sehr effizient, da insbesondere durch adenovirale Vektoren eine große Zahl verschiedener Zellen transduziert werden können. Bei Adenovirus-Infektion wird die DNA allerdings nicht in das Wirtsgenom integriert, sondern liegt episomal vor. Zudem kann eine zelluläre Immunreaktion ausgelöst werden, die die Fremdgenexpression begrenzt (Li et al., 1993 Hum Gene Ther. 4: 403-409). Eine Verminderung dieser Reaktion ist durch Vektoren möglich, die die Adenovirus E3-Region nicht deletiert haben (Ilan et al., 1997. Proc. Natl. Acad. Sci. USA 94: 2587-2592). Diese Modifikationen erlauben eine transiente, über Monate anhaltende Expression des therapeutischen Gens. Eine permanente Wirkung kann mit Adenovirusvektoren jedoch nicht erreicht werden, da wie oben erwähnt diese Vektoren nicht stabil ins Genom der Wirtszelle integrieren.

Im Gegensatz dazu sind retrovirale Vektoren in der Lage, Fremdgene stabil in das Genom von Wirtszellen zu integrieren. Retroviren gehören zu den wenigen derzeit bekannten Viren, die Mechanismen zur Integration von Fremd-DNA in das Wirtszellgenom besitzen, und diese daher dauerhaft transformieren können. Aufgrund dieser Eigenschaft werden sie häufig bei Gentherapie-Ansätzen verwendet. Bei bisher angewandten Techniken werden dazu Fremdgene in die provirale DNA von Retroviren kloniert, wobei jedoch replikations-defiziente Retroviren eingesetzt werden, die mindestens eines der für die Verpackungsfunktion kodierenden retroviralen Gene (gag-pol oder env) inaktiviert oder deletiert haben. Diese Konstrukte enthalten das Verpackungssignal Psi und die flankierenden Long Terminal Repeat-(LTR)-Sequenzen des Provirus. Diese Transkriptionseinheiten werden mit Plasmidvektoren durch herkömmliche Transfektionstechniken in die Zellen gebracht und im Zellkern üblicherweise unter der Kontrolle des LTR-Promotors in RNA transkribiert. Solche RNA wird aufgrund des Verpakkungssignals Psi als genomische virale RNA akzeptiert und in retrovirale Partikel verpackt.

Zur Bildung von infektiösen retroviralen Partikeln müssen, im einfachsten Fall, die Genprodukte gag-pol und env vorhanden sein. Diese Gene können auch ohne das Verpackungssignal über Plasmid-Transfektion in die Zellen eingeschleust oder über ein Helfervirus zur Verfügung gestellt werden. Auch können zur Herstellung von retroviralen Partikeln Zellinien, die die viralen Gene für die Verpackungskomponenten gag, pol und/oder env stabil integriert haben und exprimieren, transduziert werden. Solche Zellinien werden als "packaging"-Linien bezeichnet und sind beispielweise in der WO 97/35996 beschrieben.

Es wurden auch Plasmidkonstrukte beschrieben, sogenannte "Plasmoviren" , die alle notwendigen Komponenten für die Bildung von nicht-replizierenden retroviralen Partikeln kodieren (Noguiez-Hellin et al. 1996, Proc. Natl. Acad. Sci. USA 93: 4175-4180).

Die Verwendung von replikations-defizienten Retroviren verhindert jedoch nicht vollkommen das Risiko, daß die retrovirale DNA mit den Helfergenen rekombiniert, wodurch es zur Produktion von replikationskompetenten Viren kommen kann, die eventuell ein tumorigenes Potential besitzen.

Obwohl die Transformation von Zellen mit retroviralen Vektoren in vitro sehr effizient ist, stößt man bei gentherapeutischen Anwendungen derzeit auf Schwierigkeiten. So können Transfektionstechniken, wie sie bei der Herstellung von retroviralen Vektoren über Plasmidkonstrukte notwendig sind, nur in vitro durchgeführt werden. Direkte Verabreichung der Plasmidkonstrukte zur Produktion der defekten retroviralen Partikel im Körper ist extrem uneffizient. Ebenso lassen sich retrovirale Partikel *in vitro* nur zu vergleichsweise niedrigen Titern produzieren. Ein Aufkonzentrieren der Partikel ist durch ihre Instabilität auch nur begrenzt möglich. Die niedrigen Titer und die Instabilität von retroviralen Partikeln verhindern daher oftmals die effiziente Transformation in vivo. Methoden, wie die direkte Injektion der Partikel in das Zielorgan, oder nach Gewebsentnahme, die ex vivo-Transformation und Implantation transformierter Zellen, werden derzeit versucht. Auch ist die zielgerichtete Transduktion von bestimmten Zielorganen, das sogenannte "targeting" bei retroviralen Vektoren sehr schwierig. Es wurde versucht, durch heterologe Hüllproteine den Tropismus der Partikel zu verändern (pseudotyping).

Die verbreiteten retroviralen Vektoren auf Basis von einfachen Retroviren, wie z.B. Moloney Murine Leukemia Virus (MLV), können nur Zellen in der Teilungsphase transduzieren. Das Zellwachstum in vielen ausgewachsenen Organen ist jedoch sehr gering. In Versuchen zur hepatischen Gentherapie versuchte man daher, unter anderem durch eine teilweise Entfernung der Leber wachsendes und damit transformierbares Gewebe zu erhalten. Es wurden ebenfalls retrovirale Vektoren auf Basis von komplexen retroviralen Viren, wie z.B. HIV (lentivirale Vektoren), konstruiert, da diese auch nicht-teilende Zellen transformieren können (Reiser et al, 1996. Proc. Natl. Acad. Sci. USA 93: 15266-15271).

Um bei der Gentherapie die defekten Gene zu korrigieren, ist insbesondere eine effiziente Einschleusung der entsprechenden Gene sowie eine Langzeit-Expression der Gene notwendig. Neue Ansätze zur Herstellung von Vektoren für die stabile *in vivo*-Transduktion nutzen daher das Prinzip, Viren als Träger retroviraler Defektpartikel zu verwenden. Dabei wird insbesondere das große Insertionspotential bei DNA-Viren, wie Herpesvirus und Adenovirus, ausgenutzt. Der am weitesten fortgeschrittene Ansatz chimärer Virussysteme ist die Erzeugung defekter retroviraler Partikel durch Ko-Infektion von zwei rekombinanten Adenoviren, die für den retroviralen Vektor bzw. für die Verpackungsfunktion kodieren, wobei über das Adenovektorsystem der retrovirale Vektor und die Verpackungsproteine in die Zellen eingeschleust werden, wodurch diese Zellen zu transienten retroviralen Produktionszellen werden und die dabei gebildeten retroviralen Vektorpartikel die Nachbarzellen infizieren können (Feng et al., 1997.

Nat. Biotech. 15:866-870, Bilbao et al., 1997. FASEB J. 11:624-634).

Dieses System hat allerdings mehrere entscheidende Nachteile: Die Aufnahmekapazität von Fremdgenen von Adenovirus-Vektoren ist auf wenige Kilobasen begrenzt, sodaß es schwierig ist, alle Funktionen zur Herstellung eines defekten Retrovirus (Verpakkungskomponenten und retrovirale Vektorkomponenten) in einem Adenovirus zu vereinigen. Ein solches Konstrukt ist jedoch die Voraussetzung für eine effiziente *in vivo*-Gentherapie. Zudem werden sowohl Adenovirus als auch retrovirale Gene und Genome im Zellkern der Wirtszelle transkribiert bzw. repliziert. Diese topographische Nähe macht Rekombination zu Wildtyp-Retroviren wahrscheinlich. Prinzipiell erlaubt dieses System auch keine Deletion wichtiger retroviraler Transkriptions-Kontrollregionen, da diese für die Transkription im Zellkern nötig sind. Somit kann das Adenovirus/Retrovirus-System sicherheitstechnisch nicht im gewünschten Maße, attenuiert werden. Dasselbe gilt für das Herpes Simplex-Amplikonsystem; dieses wurde bisher nur für die Expression retroviraler Strukturproteine beschrieben, wobei Zellen, enthaltend ein lacZ-Provirus mit einem HSV-Amplikon-Vektor, enthaltend die Verpackungskomponenten, infiziert und so retrovirale lacZ-Partikel erhalten wurden (Savard et al., 1997. J. Virol. 71:4111-4117).

Ein Nachteil sowohl bei adenoviralen als auch retroviralen Vektoren ist insbesondere, daß Introns zur Verbesserung der Fremdgenexpression oder zur Stabilisierung von Vektor-RNAs in der transduzierten Zelle nicht angewandt werden können, da diese Introns bereits bei der Vektorproduktion durch zellkern-spezifische Splice-Mechanismen entfernt werden.

Für einen viralen Virusvektor als chimären Retrovirus-Carrier müssen auch Splicing-und Polyadenylierungs-Signale am richtigen Ort im retroviralen Defektvirusgenom sitzen, um nicht während der Transkription der zu transduzierenden retroviralen Genome zu Fehlsplicing bzw. vorzeitigen Kettenabbruch zu führen. Wird beispielsweise das nukleäre Polyadenylierungssignal vor den zweiten, stromabwärts gelegenen retroviralen LTR-Promotor gesetzt, so führt dies im Zellkern zu Kettenabbruch der Transkripte und zu einem nicht weiter zur Transduktion fähigen retroviralen Defektgenom. Die im Zellkern replizierenden und transkribierenden Adeno- oder Herpesviren würden im Falle der atopischen Präsenz dieser Signale kein transduzierendes Defektretrovirus bilden und können auf diese Weise nicht sicherer gemacht werden. Die Insertion retroviraler LTRs in Herpesvirus kann zudem aus nicht-oncogenen Herpesviren oncogene Subspecies hervorbringen (Isfort et al., 1992, Proc.Natl.Acad.Sci., USA 89:991-995). Dies ist insbesondere deshalb möglich, weil der Lebenszyklus der Herpesviren im Zellkern abläuft.

Auch Alphaviren wurden als Vektoren zur Produktion von Retroviren verwendet. Koinfekte mehrerer Semliki Forest-Vektoren, die über in vitro synthetisierte RNAs erhalten wurden, resultierten in infektiösen retroviralen Vektor-Partikeln (Li et al., 1993, Hum.Gene Ther. 4:403-409). Der natürliche Tropismus von Träger-Viren könnte dabei für den Gentransfer in die jeweiligen bevorzugten Zelltypen und Gewebe genutzt werden.

Aufgabe der vorliegenden Erfindung war es, ein Vektorsystem zur Verfügung zu stellen, das die oben beschriebenen Nachteile nicht aufweist und eine effiziente Bildung von retroviralen Partikeln erlaubt.

Die Aufgabe wird erfindungsgemäß durch das Zurverfügungstellen eines chimären Poxvirus gelöst, das alle Sequenzen eines defekten retroviralen Genoms, die für die Expression des retroviralen Genoms notwendig sind, enthält, wobei eine Fremdsequenz unter der transkriptionellen Kontrolle eines zellkern-aktiven Promotors in das retrovirale Genom inseriert ist.

Unter "Vektorkomponente" wird im vorliegenden Zusammenhang ein defektes retrovirales Vektorgenom, das alle für die Expression des retroviralen Genoms notwendigen Sequenzen, einschließlich des Verpackungssignals psi, sowie die für ein Fremdprotein kodierenden Sequenzen, enthält, verstanden. Die Sequenz für die Vektorkomponente umfasst im erfindungsgemäßen chimären Poxvirus insbesondere ein modifiziertes retrovirales Genom, bei dem ein Fremdgen, insbesondere eine (oder mehrere) für ein Fremdprotein kodierende Sequenz(en), Antisense-DNA, ein (oder mehrere) Ribozym(e), unter der transkriptionellen Kontrolle eines Zellkern-aktiven Promotors, insbesondere eines β-Actin-, CMV- oder SV40-early-Promotors, steht.

Das Fremdprotein kann jedes beliebige Protein sein, wobei jedoch ein Protein für die Substitutionstherapie oder Tumortherapie besonders bevorzugt ist. Für die Substitutionstherapie geeignete Proteine können dabei Plasmaproteine, wie beispielsweise Faktor II, Faktor V, Faktor VII, Faktor VIII, Faktor IX, Faktor X, Faktor XI, Faktor XIII, Protein C, Protein S, von Willebrand-Faktor oder Erythropoetin sein. Für die Tumortherapie geeignete Proteine sind Tumorsuppressorproteine wie p53 oder p73, oder "suizide Gene" wie HSV TK, Immunstimulatoren wie etwa B7.

Der Poxvirus-, insbesondere der Vaccinia-Virus-Transkriptionsapparat erkennt weder den LTR-Promotor der retroviralen Provirus-DNA als Promotor, noch werden die proviralen RNA-Prozessierungssignale erkannt. Daher werden gemäß einem besonderen Aspekt der vorliegenden Erfindung die Sequenzen der Vektorkomponente unter die transkriptionelle Kontrolle eines Poxvirus-spezifischen Promotors gestellt.

Besonders bevorzugte Promotoren sind dabei Poxvirus-Promotoren, die die Expression der frühen Gene steuern. Beim erfindungsgemäß chimären Vaccinia-Virus (RetroVac-Vektor) sind dies insbesondere die frühen Promotoren, die sowohl natürliche als auch synthetische Promotoren umfassen, wie sie beispielsweise von Davison et al. (1989, J. Mol. Bio. 210:749-769) und Pfleiderer et al. (1995, Protein Expr. Purif. 6:559-569) beschrieben sind.

Um solche Konstrukte herzustellen, kann beispielsweise der U3-Bereich vom 5'-Ende der proviralen DNA deletiert werden und durch einen Poxvirus-spezifischen Promotor ersetzt werden. Ebenso kann der U5-Bereich vom 3'-Ende der proviralen DNA deletiert werden und ein TTTTTNT-Signal am Ende der "R" -Region hinzugefügt werden. Die repetierten Bereiche "R" an den Enden der Transkripte sind für die reverse Transkription und damit für die Funktionalität der Vektorpartikel essentiell. Initiation stromaufwärts des normalen Transkriptionsstarts führt zur Synthese eines Transkriptes mit einem nicht-repetierten 5'-Ende, und damit erwartungsgemäß zu reduzierten Vektor-Titern. Die Initiationsstelle des im Prototyp-Konstrukt jeweils eingesetzten Poxvirus-Promotors sollte daher im Hinblick auf ein korrektes 5'-Ende der RNA in jedem Fall optimiert werden. Die Optimierung solcher Konstrukte liegt im allgemeinen Fachwissen und kann ohne großen Aufwand durchgeführt werden.

Mit dem erfindungsgemäßen chimären Poxvirus lassen sich in einfacher Weise durch Infektion von geeigneten Packaging-Zellinien, wie sie beispielsweise in der WO 97/35996 beschrieben sind und die die Verpackungskomponenten exprimieren, defekte retrovirale Partikel herstellen.

Gemäß einer besonderen Ausführungsform der vorliegenden Erfindung enthält das chimäre Poxvirus, die für die Vektorkomponente und die Verpackungskomponenten kodierenden Sequenzen.

Das erfindungsgemäße chimäre Poxvirus, enthaltend sowohl die Vektor- als auch die Verpackungskomponente, ist in der Lage, rekombinante defekte Retroviren in situ freizusetzen, da es in der Zielzelle sowohl die Gene für die Verpackungskomponenten, als auch eine Transkriptionseinheit, die ein retrovirales Defektgenom codiert, exprimiert. Vorzugsweise enthält das retrovirale Defektgenom, neben retroviralen Replikations- und Packaging-Signalen, das zu transduzierende und exprimierende Fremdgen, das entweder vom Promotor des retroviralen Long Terminal Repeat (LTR) selbst oder von einem zweiten Promotor kontrolliert wird.

Unter "Verpackungskomponente" werden in der vorliegenden Erfindung alle zur Bildung eines retroviralen Vektors notwendigen Gene, wie gag-pol und env, eines Retrovirus verstanden. Für die Konstruktion der erfindungsgemäßen chimären Poxviren (sogenannte Retrovac-Vektoren) können simple Retroviren wie MLV ebenso wie komplexe Retroviren, z.B. humanes Immunodeficiency Virus (HIV), verwendet werden. Ebenso können zur Veränderung des Wirtsspektrums heterologe Hüllproteine (z.B. VSV-G) für die transformierenden retroviralen Partikel exprimiert werden, wie bereits für retrovirale Vektoren beschrieben wurde. Generell lassen sich die meisten Weiterentwicklungen an retroviralen Vektoren auch auf RetroVac-Vektoren anwenden. Das schließt auch die Expression von Fremdgenen unter Kontrolle von gewebsspezifischen Promotoren und Vektoren zur "site-specific" -Integration von Fremd-DNA mit ein.

Erfindungsgemäß stehen die gag-, pol- und env-Gene vorzugsweise unter der Kontrolle eines Poxvirus-Promotors. Bevorzugt sind dabei Promotoren mit einem starken frühen Anteil, in Übereinstimmung mit der Expressionscharakteristik des Poxvirus. Die für die Verpackungskomponente kodierenden Sequenzen gag/pol und env können dabei in einer Transkriptionseinheit oder auf getrennten Transkriptionseinheiten unter der Kontrolle eines Poxvirus/Vaccinia-Virus spezifischen Promotors exprimiert werden, wobei die Transkriptionseinheit in einer essentiellen oder nicht-essentiellen Region des Poxvirusgenoms integriert sein kann.

Bei Poxvirus-Transkripten findet kein "splicing" statt. Retrovirale Gene, bei deren Expression splicing beteiligt ist, werden daher gemäß einem weiteren Aspekt der vorliegenden Erfindung als Intron-freie Leseraster in das erfindungsgemäße chimäre Poxvirus kloniert. Das betrifft je nach dem gewählten retroviralen System die Gene env, tat, ref oder andere.

Mit dem erfindungsgemäßen chimären Virus lassen sich in einfacher Weise sowohl *in vitro* als auch in vivo defekte retrovirale Partikel herstellen. Bisher wurde noch kein System beschrieben, bei dem alle Komponenten für ein defektes retrovirales Partikel auf einem einzigen Träger-Virus kodiert und kombiniert sind. Dies ist eine wesentliche Voraussetzung für die effiziente Anwendung solcher Vektoren *in vivo.*

Die Entwicklung von "Packagingvektoren" und Delivery/Amplifikations-Systemen transduzierender Defekt-Retroviren auf Basis von chimären Poxviren wurde bisher nicht beschrieben. Beschrieben wurde jedoch die Verwendung von Poxvirusvektoren zur Expression retroviraler Komponenten, insbesondere von gag-pol und env von HIV in Poxvirusvektoren (Moss, 1996, Proc. Natl. Acad. Sci., USA 93: 11341-11348, Paoletti, 1996, Proc. Natl. Acad. Sci., USA 93: 11349-11353). Zweck dieser Studien waren jedoch virologische Grundlagenforschung und Impfstoffentwicklung. So ist z.B. bekannt, daß die Expression des HIV-1 gag-pol Leserasters in VV (Vaccinia-Virus) zur Bildung von Pseudopartikeln führt (Karacostas et al., 1989, Proc. Natl. Acad. Sci., USA 86:8964-8967). Ebenso führt die Koexpression von gag und env zur Bildung HIV-ähnlicher Partikel, die insbesondere zur Impfstoff-Entwicklung geeignet sind (Haffar et al., 1990, J. Virol. 64: 2653-2659). Auch die Doppel-Expression von gag-pol und env in Poxvirus-Vektoren als SIV Kandidat-Vakzine ist beschrieben (Hirsch et al., 1996, J. Virol. 70: 3741-3752).

Als Vektoren für die erfindungsgemäßen chimären Poxviren werden insbesondere Chordopoxviren, die auch Viren aus der Gruppe der Orthopoxviren und der Avipoxviren einschließen (Moss, 1996, Poxviridae: the viruses and their replication. In: Fields et al. (ed.) Fields Virology. Third Edition (3^{rd}.Ed.) Vol. 2. Lippincott-Raven, Philadelphia, 2637-2671), eingesetzt. Es werden jedoch vorzugsweise solche Poxviren eingesetzt, die Säugerzellen infizieren, sich in diesen jedoch nicht vermehren (nichtreplizierende Vektoren). Als Vektoren werden daher bevorzugterweise *Vaccinia-Viren* verwendet, insbesondere attenuierte *Vaccinia-Viren* (Paoletti, 1996, Proc. Natl. Acad. Sci., USA 93: 11349-11353), Modified Vaccinia Ankara (MVA), oder defekte Vaccinia-Viren, wie etwa in der WO 95/30018 und in Holzer et al. (1997, J. Virol., 71:4997-5002) beschrieben. Der letztgenannte defekte Virus-Vektor läßt sich leicht zu Titern von 10⁸ plaque forming units (PFU)/ml vermehren und zu Titern von 10¹¹ PFU/ml aufkonzentrieren. Ausserdem verharren etwa D4-deletierte Defektviren in der frühen Phase der Replikation, was zu einer anhaltenden Synthese früher RNAs führt. Nur frühe Vaccinia-Virus (VV)-RNAs haben definierte 5'- und 3'-Enden, was für die Synthese funktionaler retroviraler Genome eine grundsätzliche Voraussetzung ist. Ein Vaccinia-Virus-Defektpartikel, das die gesamte genetische Information für einen retroviralen Vektor exprimiert, bewirkt in der infizierten Zelle die Bildung von nicht-replizierenden retroviralen Partikeln, die ihrerseits transformierendes Potential haben. Da sich, wie oben erwähnt, insbesondere Vaccinia-Viren zu Titern von 10¹¹ PFU/ml aufkonzentrieren lassen, liegt der Titer bezogen auf die Transformation (ausgedrückt in CFU/ml) damit höher als herkömmliche Retrovirustiter (>10⁶-10⁷). Durch die Expression von retroviralen Partikel *in situ* sind ausserdem sämtliche über die Zeit freigesetzten transformierenden Partikel für die Transduktion relevant und nicht, wie bei in vitro-Produktion von Retroviren, die transduzierenden Partikel pro Volumen Zellkulturüberstand.

Gemäß einer besonderen Ausführungsform der vorliegenden Erfindung dient ein nicht replizierendes Vaccinia-Virus, wie etwa in der WO 95/30018 beschrieben, als Träger der genetischen Information für nicht,replizierende defekte Retroviren. Sowohl das modifizierte retrovirale Genom, das das Fremdgen und das Verpakkungssignal Psi enthält, als auch die retroviralen Gene gag-pol und env werden auf dem Genom eines defekten Vaccinia-Virus codiert. Die für die Verpackungskomponente kodierenden Sequenzen gag/pol und env können in einer Transkriptionseinheit oder mehreren Transkriptionseinheiten unter der Kontrolle eines Poxvirus/Vaccinia-Virus-spezifischen Promotors exprimiert werden und in einer essentiellen oder nicht-essentiellen Region des Poxvirusgenoms integriert sein. Die Sequenzen der Vektorkomponente können als eigenständige Transkriptionseinheit, ebenfalls unter der Kontrolle eines Poxvirus, vorzugsweise eines frühen Poxvirus-Promotors, in einer essentiellen oder nicht-essentiellen Region des rekombinanten Virus inseriert sein, wobei die Insertion in eine essentielle Region bevorzugt ist.

Die DNA-Sequenz TTTTTNT führt zu einem Abbruch der frühen Poxvirus/Vaccinia-Transkription. Um die retroviralen Sequenzen effizient zu exprimieren, sollten daher in den retroviralen Sequenzen bzw. im Fremdgen möglicherweise vorhandene TTTTTNT-Signale durch Punktmutationen abgewandelt werden, ohne den Aminosäurekontext der retroviralen Proteine zu verändern oder Steuerungssignale auf dem RNA-Genom der defekten Retroviren, wie z.B. Psi und die Integrationsregionen "att", zu zerstören.

Gemäß einer besonderen Ausführungsform der vorliegenden Erfindung weisen daher die für die Verpackungs- bzw. Vektorkomponenten kodierenden Sequenzen keine Poxvirus/Vaccinia-Virus-spezifischen Stopsignale auf.

Ebenso wie für die Expression der Vektorkomponente-Sequenz ist es im erfindungsgemäßen chimären Poxvirus-RetroVac-System bevorzugt, die Vektorkomponente-Sequenz unter die transkriptionelle Kontrolle eines Poxvirus-spezifischen Promotors zu stellen.

Bei den in der vorliegenden Erfindung beschriebenen bevorzugten chimären Defekt-Vaccinia-Virus-Konstrukten wurde stromabwärts der 3'-Region ein Signal für die Termination früher Vaccinia-Virus-Transkripte eingeführt, womit sich das 3'-Ende der entstehenden mRNAs von dem bisher beschriebener retroviraler Vektorgenome unterscheidet. Nur die sogenannten frühen Vaccinia-mRNAs haben mehrheitlich ein definiertes 3'-Ende. Späte Vaccinia-mRNAs, die den überwiegenden Anteil der viralen Transkripte darstellen, terminieren nicht an konkreten Signalen, haben heterogene Längen und sind daher als retrovirale Vektorgenome ungeeignet. D4-defekte *VV* treten nicht in die späte Phase der Replikation ein. Dies konnte durch ³⁵S-Markierungsexperimente von Proteinen und in Northernblot-Experimenten durch eine unerwartet lang anhaltende frühe Expression gezeigt werden (Holzer et al., 1997, J. Virol. 71:4997-5002). Damit erwiesen sie sich als ein unter Poxviren einzigartiges Werkzeug zur Synthese definierter retroviraler Genome.

Ein Vorteil der erfindungsgemäßen chimären Poxviren ist es, daß sie sich zu sehr hohen Virustitern vermehren und aufkonzentrieren lassen. Zudem sind die von Poxvirus, insbesondere Vaccinia-Virus, abgeleiteten Vektoren außerordentlich stabil, infizieren effizient Organe, wie Leber oder Milz und produzieren direkt im Zielorgan transformierende, aber nicht replizierende retrovirale Partikel (in vivo-Amplifikation der retroviralen Partikel).

Da Poxviren gewebsspezifisch sind (primär affine Organe), findet eine Transformation durch den erfindungsgemäßen chimären Poxvirus, insbesondere des chimären Vaccinia-Virus, gewebsspezifisch statt. Bespielsweise findet die Infektion mit chimären *Vaccinia-Virus* (Retrovac-Hybridvektoren) bevorzugt in jenen Geweben statt, die sowohl dem Tropismus von Vaccinia-Virus entsprechen, als auch die Rezeptoren für die eingesetzten retroviralen Hüllproteine exprimieren. Über geeignete Kombination des verwendeten Vaccinia-Virus-Stammes und pseudotyping läßt sich sowohl ein stringenteres "targeting" des therapeutischen Gens als der retroviralen Vektoren erreichen.

Die chimären Poxvirus-Vektoren, insbesondere die auf defekten Vaccinia-Virus basierenden Retrovac-Vektoren, vereinen die Fähigkeit von Retroviren, Fremd-DNA stabil in Zielzellen integrieren zu können, mit den technischen Vorteilen von Poxvirus/Vaccinia-Vektoren. Ein Hauptmerkmal des Systems ist, daß bei Infektion einer Wirtszelle durch das chimäre Poxvirus, insbesondere eines auf Vaccinia-Virus-Vektor basierenden RetroVac-Vektors, die für die Bildung von funktionalen retroviralen Partikeln notwendigen Proteine, exprimiert und die Fremdgene enthaltenden mRNAs transkribiert und als genomische RNA in retrovirale Partikel verpackt werden. Diese Partikel sind nicht vermehrungsfähig (replikations-defizient), haben aber transformierendes Potential. Während die primär mit dem chimären Poxvirus, insbesondere dem chimären Vaccinia-Virus, infizierten Zellen absterben, führt die sekundäre retrovirale Infektion weiterer Zellen durch die gebildeten retroviralen Partikel zur permanenten Integration der retroviralen Sequenzen und damit der für das Fremdgen kodierenden Sequenzen in das Zellgenom.

Das Freisetzen defekter retroviraler Partikel durch die erfindungsgemäßen chimären Poxviren, war insofern überraschend, da normalerweise die Transkription von Retroviren sowie das "capping" der genomischen RNAs im Zellkern stattfindet (Coffin, 1996, Retroviridae: the viruses and their replication. In: Fields et al. (ed.) Fields Virology. Third Edition (3^{rd}. Ed.) Vol. 2, Lippincott-Raven, Philadelphia, 1767-1847). Im gezeigten chimären Poxvirus-System finden Transkription und Capping im Cytoplasma statt. Die Vaccinia-Virus-spezifischen Cap-Strukturen sind daher kein Hindernis für ein retrovirales Packaging. Es zeigte sich überraschenderweise, daß vom cytoplasmatischen Poxvirus/Vaccinia-Transkriptionssystem generierte Transkripte kompatibel sind mit dem retroviralen Transkriptions- und Replikationssystem. Dies war insofern unerwartet, als sich die Polyadenylierungssignale, die im Zellkern erkannt werden, innerhalb der U3- und der R-Region des Provirus befinden.

Poxviren sind in der Regel lytische Viren, wodurch Zellen, die mit einem chimären Poxvirus infiziert wurden, meist absterben. Durch die so verursachten Läsionen im Gewebe kann die Teilung von benachbarten Zellen bewirkt werden, die dadurch für retrovirale Transformation durch die gebildeten retroviralen Partikel noch empfänglicher werden. Dieser Proliferationseffekt wird, im Falle ektodermaler Zellen durch Poxvirus-eigene Wachstumsfaktoren verstärkt.

In einer besonderen Ausführungsform weist daher das erfindungsgemäße chimäre Poxvirus Sequenzen kodierend für einen Wachstumsfaktor oder ein Mitogen auf. Dadurch kann der Proliferationseffekt der Nachbarzellen der Poxvirus-infizierten Zellen durch Expression vom chimären Poxvirus verstärkt werden.

In einer weiteren besonderen Ausführungsform kann man Retrovac-Vektoren auf der Basis von Lentiviren konstruieren, bei denen allerdings akzessorische Lentivirus-Gene im VV-Carrier exprimiert werden müssen, die dem System die gewünschten Eigenschaften verleihen.

Im Gegensatz zu Plasmidtransfektion, chimären Adenovirus/ Retrovirus-Vektoren oder chimären Herpesvirus/Retrovirus-Vektoren findet die Expression durch Vaccinia-Virus im Cytoplasma der Wirtszelle statt. Während nach Plasmidtransfektion oder bei Adenovirus/Retrovirus-Infektion die Transkription der Fremdgene im Zellkern mit Hilfe des zellulären Transkriptionsapparates stattfindet, erfolgt die Genexpression von Vaccinia-Virus ausschließlich im Cytosol durch einen viralen, vom zellulären verschiedenen Transkriptionsapparat. Dies stellt insofern einen entscheidenden Sicherheitsvorteil des erfindungsgemäßen Poxvirus-Vektors dar, da dadurch die Generation replizierender Retroviren sehr unwahrscheinlich wird. Dieser Ansatz erlaubt erstmals, nukleare Transkriptionssignale auf dem retroviralen Genom ausschließlich unter dem Gesichtspunkt der optimalen Fremdgenexpression und der Sicherheit in der transduzierten Zielzelle anzuordnen, da die Transkription genomischer retroviraler RNA durch Vaccinia-Virus unabhängig vom Zellkern der Zielzelle erfolgt. Ebenso können Introns zur Verbesserung der Expression eines therapeutischen Gens eingesetzt werden, da diese die Vektor-RNA Expression im Vaccinia-Virus-System nicht beeinflussen.

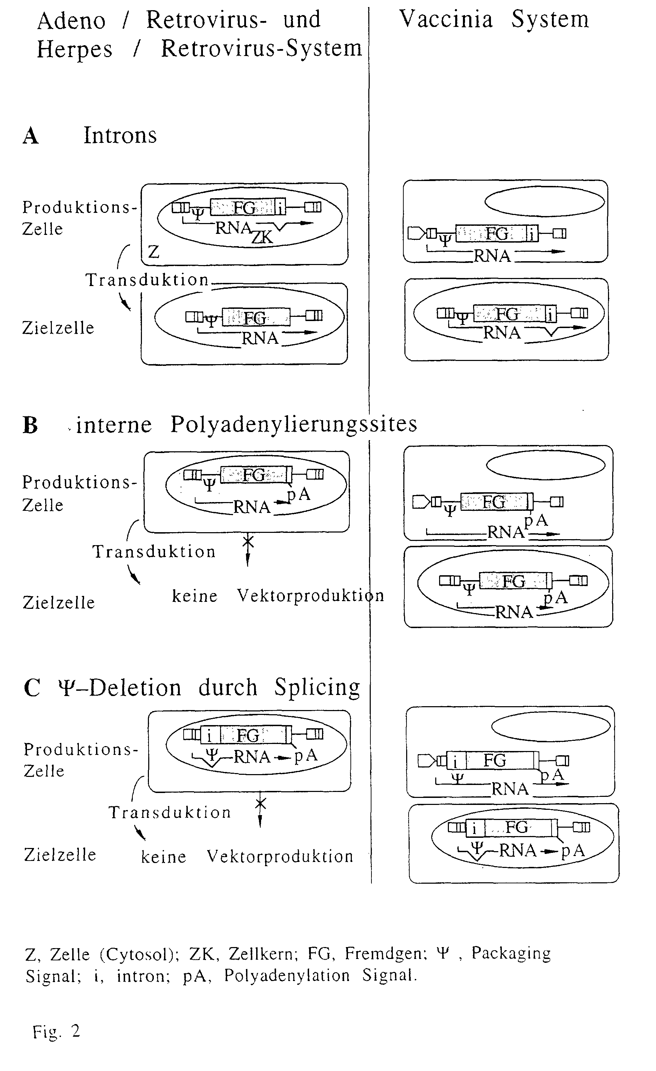

Ein weiterer besonderer Vorteil des erfindungsgemäßen Virusvektor-Systems basiert auf einem im Cytoplasma replizierenden DNA-Virus ist, daß es, im Gegensatz zu sich im Kern vermehrenden Viren, retrovirale Transkriptionseinheiten Transkriptionssignale enthalten können, die normalerweise im Kernzusammenhang nicht erlaubt bzw. nicht möglich sind, da im Lebenszyklus eines cytoplasmatisch transkribierenden Virus-Vektors kein Splicing stattfindet. So kann im Retrovac-System z.B. das retrovirale Packaging-Signal Psi durch Splicing-Signale flankiert werden, was nach Transduktion der retroviralen Defektgenome und Transkription derselben im Wirt zu RNAs führt, die keine Packaging-Signale haben und somit ein wesentliches Merkmal retroviraler Genome verloren haben (siehe Fig. 2 C). Diese Möglichkeit führt zu einer wesentlichen Steigerung der Sicherheit des Systems der im Poxvirus-System produzierten retroviralen Defektgenome. Die Funktionalität des Packaging-Signals außerhalb des Wildtyp-Kontextes wurde bereits gezeigt.

Ein weiterer Aspekt der vorliegenden Erfindung ist, daß bei cytoplasmatischer Transkription der retroviralen Defekt-RNAs, bewährte Transkriptionseinheiten mit Intron-Exon-Struktur unbeschadet (ohne Splicing) über retrovirale Transduktion ins Genom des Wirts integrierbar sind (siehe Fig. 2A). Bei der Etablierung permanent exprimierender Zellinien werden in der Regel Genkassetten transferiert, die aus Promotor, Open Reading Frame (ORF) des Fremdgens, Intron und Polyadenylierungssignal, kloniert in ein bakterielles Plasmid, bestehen. Für eine optimale mRNA-Produktion sind Introns in Transkriptionseinheiten nötig, insbesondere wurde dies auch in transgenen Tieren beobachtet. Die meisten kommerziell erhältlichen Expressionsvektoren für höhere Zellen enthalten innerhalb ihrer Expressionskassetten empirisch ermittelte Promotor-Intron-Kombinationen; bewährt hat sich z.B. die CMV-Promotor/Enhancer-SV40-Intron-Kombination im Vektor pCMVβ (Clontech Laboratories, Palo Alto). Derartige optimierte Einheiten mit Intron-Exon-Struktur lassen sich über die bisher bekannten retroviralen bzw. chimären Gentransfer-Vektoren nicht transduzieren.

Das erfindungsgemäße Retrovac-System löst dieses Problem, da aufgrund des fehlenden Splice-Apparates in Vaccinia-Virus keine Introns während der Transkription entfernt werden. Nach Transduktion der im Retrovac-System produzierten retroviralen Defektgenome kann daher die komplette Transkriptionseinheit in der Zielzelle integriert werden (siehe Fig. 2A). Dies ist insbesondere beim Transfer zum Zweck der Gentherapie schwierig zu exprimierender cDNAs, wie die des Gerinnungsfaktors VIII, wichtig und erlaubt den Transfer optimierter Promotor/Intron-Kombinationen.

Gemäß einem besonderen Aspekt der Erfindung werden daher durch das erfindungsgemäße System defekte retrovirale Partikel enthaltend ein intronhaltiges Genom, zur Verfügung gestellt.

Ein RNA-Prozessierungssignal, das an definierter Stelle nur durch cytosolische Transkriptionssysteme in die Zielzelle übertragen werden kann, ist ein internes Polyadenylierungs-Signal, das eine definierte Terminierung des Transkriptes im Zellkern bewirkt (Fig. 2B). Eine Terminierung des Transkriptes vor Fertigstellung eines kompletten retroviralen Defektgenoms im Zellkern, wie durch Transduktion im Retrovac-System produzierter retroviraler Defektgenome, erlaubt eine besonders sichere Gentherapie, da das einmal integrierte Fremdgen nicht weiter transduzierbar ist, weil sein Transkript vorzeitig terminiert. Ebenso ist nur im Retrovac-System die Lokalisierung des Packaging-Signals zwischen Splice-Signalen möglich (Fig. 2C), was in der Zielzelle zur Transkription retroviraler Defektgenome führt, denen das Packaging-Signal fehlt. Kombiniert mit internen Polyadenylierungssignalen lassen sich so besonders sichere Genkassetten übertragen.

Ein weiterer Aspekt der vorliegenden Erfindung betrifft eine Zusammensetzung enthaltend ein chimäres Poxvirus der oben beschriebenen Art und einen pharmazeutischen Träger.

Das erfindungsgemäße Retrovac-System läßt sich für alle Anwendungen einsetzen, die für die Gentherapie mit Retroviren sinnvoll sind. Es ist für die In vivo-Gentherapie zur Behandlung von Plasmaprotein-Defekten, insbesondere Hämophilien (Faktor IX- und Faktor VIII-Mangel) und Erythropoetin-Mangel geeignet. Die Verabreichung kann dabei intravenös oder intramuskulär erfolgen.

Die Stabilität der erfindungsgemäßen chimären Poxviren erlaubt bei entsprechender Formulierung auch eine orale Verabreichung der Vektoren als Sprays, was eine Inhalations-Behandlung der cystischen Fibrose ermöglicht.

Eine weitere Anwendung der RetroVac-Vektoren ist die Tumortherapie. Es hat sich beispielsweise gezeigt, daß der Transfer von sogenannten Suizid-Genen in Tumoren oder deren Metastasen sich in tierexperimentellen Modellen als erfolgversprechend erwies (Caruso et al., 1993, Proc. Natl. Acad. Sci., USA 90: 7024-7028, Culver et al., 1992, Science 256:1550-1552). Bei derartigen *ex*perimentellen Ansätzen wurde das HSV-TK-Gen, das nicht-toxische Nukleosid-Analoge (wie Ganciclovir) in toxische umwandelt, durch intratumorale Injektion von Verpackungs-Zellinien, die in situ defekte retrovirale-Partikel freisetzen, transduziert und anschließend eine Chemotherapie mit Ganciclovir durchgeführt. Mit dem erfindungsgemäßen RetroVac-Vektor können daher Suizid-Gene sicher und effizienter verabreicht werden.

Ebenso können Apoptosis-induzierende Tumorsuppressor-Gene, wie etwa das p53-Gen, das in über 50% der Tumore verändert ist, im erfindungsgemäßen RetroVac-Vektor als Fremdgen inseriert werden. Beim gentherapeutischen Transfer dieser Tumorsuppressor-Gene würden die Tumorzellen durch die endogene Kontrolle das Wachstum einstellen.

Eine ex vivo-Zelltransduktion zur Tumortherapie (beispielsweise Leukämie), ist ebenfalls möglich; desweiteren die direkte intratumorale Injektion zur Krebstherapie.

Zur Erhöhung der Sicherheit des Systems können Vektoren verwendet werden, die sogenannte Suizid-Gene tragen, wie z.B. das Herpes Simplex-Virus (HSV) mit eingebautem Thymidin-Kinase-Gen, die eine Chemotherapie erlauben würden, wenn es in vivo zur Aktivierung von Oncogenen durch den Transduktionsvorgang kommen sollte.

Das System kann auch zur Produktion permanenter Zellinien verwendet werden, da retrovirale Partikel eine besonders effiziente Transduktion erlauben und durch den Transfer von RNA-Prozessierungssignalen (Introns, Polyadenylierungssites) optimale Genkassetten transferiert werden können. Erstmals ist auf diese Weise der äußerst effiziente Transfer von Genkassetten möglich, der im Zusammenhang mit Screening-Techniken das schnelle Auffinden hochexprimierender Zellklone erlaubt.

Ein besonderer Aspekt der vorliegenden Erfindung betrifft daher die Verwendung des erfindungsgemäßen chimären Poxvirus zur Herstellung eines Arzneimittels, das insbesondere für die Gentherapie und Tumortherapie eingesetzt werden kann.

Im Rahmen der vorliegenden Erfindung wurden über die oben erwähnten chimären Poxviren defekte retrovirale Partikel erhalten, die sich insbesondere dadurch auszeichnen, daß sie noch ein intronhaltiges Genom enthalten.

Die Erfindung wird anhand der nachfolgenden Bespiele und der Zeichnungsfiguren näher erläutert, wobei sie jedoch nicht auf diese beschränkt ist.

Es zeigen:

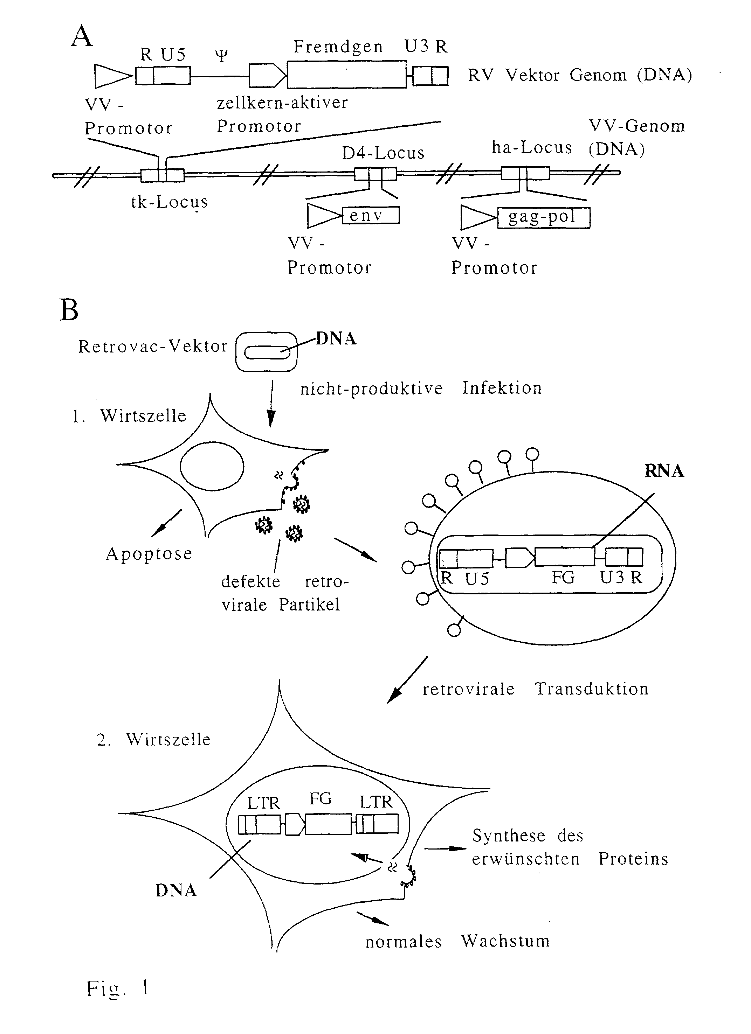

Figur 1: Die schematische Darstellung der Konstruktion von chimärem Vaccinia-Virus. Fig. 1A zeigt dabei die mögliche Insertionsposition der Verpackungs- und Vektorkomponenten, die gegebenenfalls in verschiedene Regionen des Poxvirusgenoms integriert sein können und unter der transkriptionellen Kontrolle eines Poxvirus-Promotors stehen. Fig. 1B zeigt das Infektionsschema einer Zelle mit dem erfindungsgemäßen Poxvirus und die Produktion von defekten retroviralen Partikeln, die ihrerseits wieder Zellen infizieren und in das Wirtsgenom stabil integrieren.
Figur 2: Einen Vergleich eines im Cytoplasma replizierenden chimären Poxvirus mit den System von im Kern replizierenden Adeno- bzw. Herpesviren, insbesondere im Hinblick auf den Splice-Mechanismus.
Figur 3: Das Konstruktionsschema des Plasmids pTKgpt-LSXN
Figur 4: Das Konstruktionsschema des Plasmids pD4-SX
Figur 5: Das Konstruktionsschema des Plasmids pTK-MLVg
Figur 6: Das Konstruktionsschema des Plasmids pTKgpt-VP-FIX

### Beispiel 1:

### Konstruktion des Vaccinia-Virus(VV)-Defektvirus vd-LXSN

Im retroviralen Defektvirus-Genom des proviralen Vektorplasmids pLXSN (Miller et al., 1989, Biotechniques. 7:980-990) wurden die frühen Vaccinia-Virus (VV)-Transkriptions-Stop-Signale entfernt und das so deletierte Genom hinter einen starken VV frühen Promotor kloniert und dann in den Thymidin-Kinase (tk) Locus des VV-Defektvirus-Stammes vD4-vA (Holzer et al., 1997, J. Virol. 71:4997-5002) inseriert. Im resultierenden VV-Defektvirus vd-LXSN werden dadurch im frühen VV-Infektionszyklus retrovirale Defektgenome transkribiert.

### Konstruktion des Plasmids pTKgpt-LXSN

Das retrovirale Defektgenom im Plasmid pLXSN (Clontech Laboratories, Inc., Palo Alto, CA) hat folgende Abfolge von genetischen Elementen: LTR-Psi-MCS/SV40-neo-Genkassette-LTR (LTR, long terminal repeat; Psi, packaging-Signal für retrovirale RNA; MCS, multiple cloning site; SV40-neo, SV40 Promotor-Neomycin-Resistenz-Genkassette). In die MCS dieses Konstrukts können geeignete Fremdgene inseriert werden; die SV40-neo-Genkassette dient als Selektionsmarker, mit dessen Hilfe die retroviral transduzierten Zellen selektiert werden können.

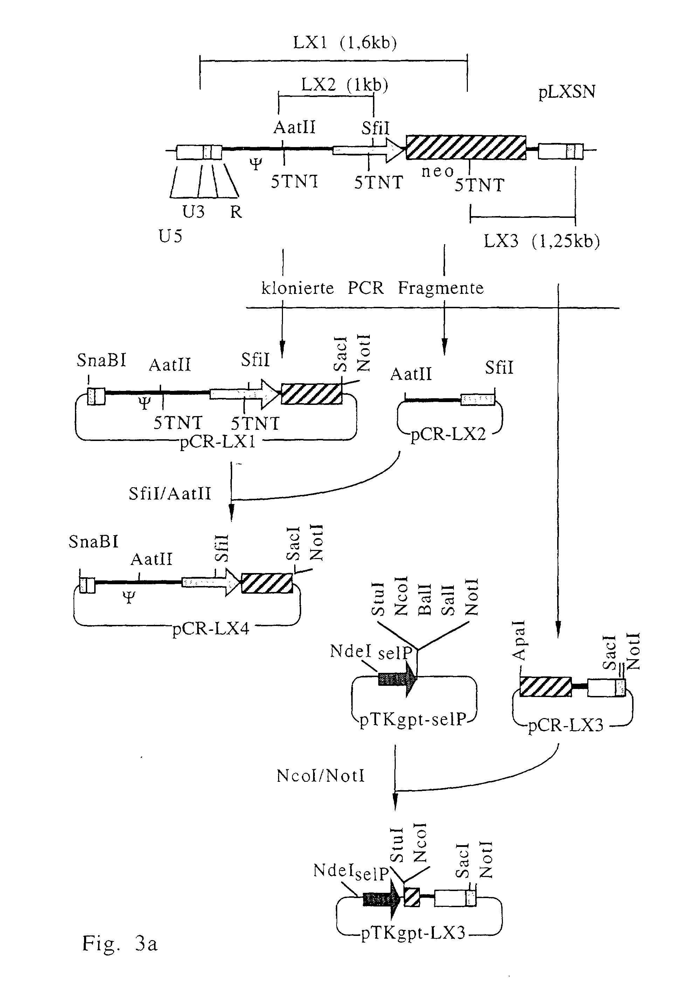

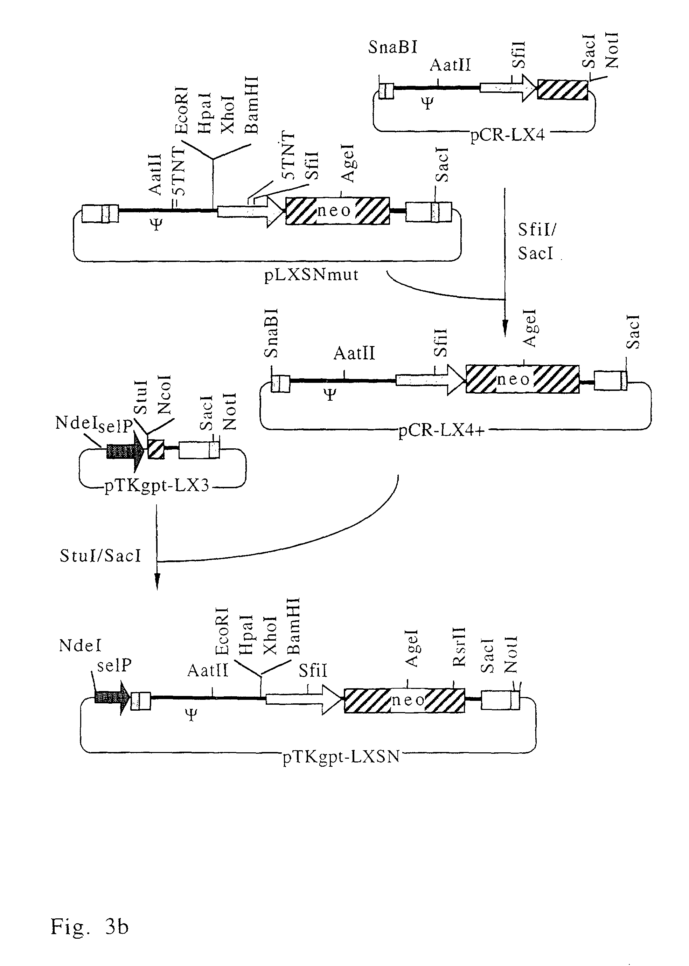

Die provirale retrovirale DNA in pLXSN hat drei TTTTTNT-Signale, die zu einem teilweisen Abbruch der frühen Transkription führen. Durch PCR wurden einzelne Fragmente kloniert und durch Mutagenese modifiziert (Fig. 3). Es wurden dazu mittels verschiedener Oligonucleotide die folgenden 3 PCR-Fragmente mit dem Plasmid pLXSN als Template amplifiziert und dabei Punktmutationen eingeführt, die vorhandene TTTTTNT-Signale abwandeln. Die Herstellung der Fragmente LX1, LX2 und LX3 erfolgte wie folgt beschrieben:

LX1: Ein 1,6kb-Fragment wurde mit den Oligonukleotidprimern oRV-5 (5'-TACGTACGGC GCGCCAGTCT TCCGATAG-3') und oRV-6 (5'-GAACCGGTCG CCCCTGCGCT GAC-3') generiert, wobei durch oRV-5 eine SnaBI-Schnittstelle und durch oRV-6 eine AgeI-Schnittstelle eingeführt wurden.

LX2: Ein 1kb-Fragment wurde mit den Oligonukleotidprimern oRV-7 (5'-AGACGTCCCA GGGACTTTGG GGGCCGTATT TGTGGC-3') und oRV-8 (5'-AGGCCGAGGC GGCCTCGGCC TCTGCATAAA TAAATAAAAT TAG-3') generiert und durch die beiden Primer das TTTTTNT-Motive durch Punktmutationen abgewandelt; oRV-7 bindet im Bereich einer AatII-Schnittstelle, oRV-8 im Bereich einer SfiI-Schnittstelle.

LX3: Das 3'-Ende des geplanten Vektorkonstrukts wurde als 1,2kb PCR-Fragment mit den Oligonukleotidprimern oRV-9 (5'-CGACCGGTTC TATTTGTCAA GACCGACCT-3')und oRV-10 (5'-GCGGCCGCAA CTGCAAGAGG GTTTATTGGA-3') generiert. oRV-10 bindet am 3'-Ende der "R" -Sequenz und führt eine NotI-Schnittstelle ein.

Um ein TTTTTNT-Signal im Neo-Leseraster abzuwandeln wurde eine Mutagenese im Plasmid pLXSN gemacht. Die Mutagenese wurde mit dem QuickChange™ Mutagenesekit der Fa. Stratagene und den Primern oRV-35 (5'-AAATAGAACC GGTCGCCCCT GCGCTGAC-3') und oRV-9 gemacht. Das resultierende Plasmid wurde mit pLXSNmut bezeichnet (Fig. 3b), und weist an der mutagenisierten Stelle eine PinAI-Schnittstelle auf.

Das LX1 PCR-Produkt wurde in den Vektor pCRII (Invitrogen) kloniert, das Plasmid, das das PCR-Produkt in der gewünschten Orientierung enthielt, selektiert und als pCR-LX1(b) bezeichnet. Das LX2 PCR-Produkt wurde mit den Enzymen AatII und SfiI geschnitten und in AatII und SfiI geschnittenes pCR-LX1(b) gesetzt. Das resultierende Plasmid war bis auf die abgewandelten TTTTTNT-Signale identisch zu pCR-LX1(b) und wurde mit pCR-LX4 bezeichnet. Die Ligation eines 1,4kb SfiI/SacI-Fragmentes aus dem Plasmid pLXSNmut in SfiI/SacI geschnittenes pCR-LX4 ergab pCR-LX4+. Dieses Plasmid enthält den gesamten transkribierten Bereich des Vektors bis auf das äußerste 3'-Ende.

Das korrekte 3'-Ende wurde als 0,8kb-Fragment aus pCR-LX3 in den VV-Rekombinationsvektor pTKgpt-selP (Pfleiderer et al., 1995, Protein Expr. Purif. 6:559-69) gesetzt. Das erhaltene Plasmid wurde mit pTKgpt-LX3 bezeichnet (Fig. 3a). Der Plasmidvektor pTKgpt-LXSN wurde durch Ausschneiden eines 2,7kb SacI/SnabI-Fragmentes aus pCR-LX4 und Klonierung in mit SacI und StuI geschnittenes pTKgpt-LX3 erhalten und enthielt unter der Kontrolle eines starken VV-Promotors den gesamten transkribierten Bereich des Vektors pLXSN vom Beginn der "R" Sequenz ( Coffin, J. M., 1996, Retroviridae: The viruses and their replication, p. 1767-1847. *In:* Fields et al. (ed) Fields, Virology, 3^{rd} ed, vol. 2. Lippincott-Raven, Philadelphia.) im 5'-LTR bis zum Ende der "R" -Sequenz im 3'-LTR, wobei die drei ursprünglich vorhandenen TTTTTNT-Motive abgeändert sind. (Fig. 3b).

### Konstruktion des VV-Defektvirus vd-LXSN

Zur Konstruktion des VV-Defektvirus vd-LXSN wurden RK-D4R-44.20-Zellen nach Standardprotokoll mit dem Defekt-*VV* vD4-vA (Holzer et al., 1997, J. Virol. 71:4997-5002) infiziert und mit dem Plasmid pTKgpt-LXSN transfiziert. Virus-Isolate wurden in RK-D4R-44.20-Zellen unter gpt-Selektion (Falkner et al., 1988, J. Virol. 62:1849-1854) Plaque-gereinigt. Das so erhaltene *VV*-Defektvirus vd-LXSN exprimiert das gesamte defekte retrovirale RNA-Genom von pLXSN. Die korrekte genomische Struktur von vd-LXSN wurde im Southernblot und über PCR nachgewiesen.

### Beispiel 2:

### Produktion von defekten Retrovirus-Partikeln mittels des VV-Defektvirus vd-LXSN in der Packaging-Zellinie PT67.

Das Defektvirus vd-LXSN enthält und transkribiert ein Retrovirus-Defektgenom (siehe Beispiel 1). Die Zellinie PT67 (erhalten von Clontech Laboratories, Inc., Palo Alto, CA) exprimiert die gag-pol und env Gene von murinem Leukämie-Virus (MLV), die zum Verpacken retroviraler Defektgenome gebraucht werden (Miller et al., 1996, J Virol. 70:5564-71.). Um die Hypothese zu überprüfen, daß defekte *VV* in Gegenwart von gag-pol- und env-Proteinen defekte Retrovirus-Partikel bilden können, wurden PT67-Zellen mit vd-LXSN infiziert. Die Kultivierung der PT67-Zellen erfolgte nach Standardbedingungen, wie in RetroXpress System User Manual. Clontech Laboratories, Inc., Palo Alto, CA, 1997 beschrieben. Der Infekt mit vd-LXSN erfolgte bei 0,05 oder 0,5 PFU (plaque forming units) pro Zelle. Zu verschiedenen Zeitpunkten (3h bis 72h nach Infektion) wurden die retrovirale Vektorpartikel in den Überständen in NIH-3T3-Zellen (ATCC CRL-1658) in Gegenwart von 500ug/ml G418 titriert (Miller et al., 1996, J. Virol. 70:5564-71). Die retroviralen Titer erreichten 6h nach *VV*-Infektion ein Maximum und waren in diesem Bereich jeweils abhängig von der vd-LXSN Infektionsdosis. In einem typischen Experiment wurden bei einer Primärinfektion von 0,5 PFU vd-LXSN pro Zelle nach 3h 10² CFU (colony forming units) und nach 6h 2x10² CFU pro ml Kulturüberstand gezählt. Kontrollexperimente mit dem Plasmid pLXSN resultierten in 10²CFU-10³ CFU pro ml 48h nach Transfektion. Die Retrovirus-Titer, die durch vdLXSN-Infektion induziert wurden, fielen zum Zeitpunkt 12h nach Infektion auf 20 CFU/ml ab um 48h bis 72h pi wieder auf maximal 2x10² CFU/ml anzusteigen. Dieser zweite Anstieg war nicht von der initialen Infektionsdosis abhängig und wird auf eine Retrovirus-vermittelte Transformation von *VV*-uninfizierten PT67 zurückgeführt. Keine retroviralen Partikel wurden nach Transfektion des Plasmids pTKgpt-LXSN in PT67 erhalten, was beweist, daß die Expression dieses Konstruktes, anders als z. B. bei Adenovirus-Carriern, nur im viralen Kontext möglich ist. Dieses Beispiel zeigt, daß defekte *VV* als Träger transduzierender retroviraler Defektgenome geeignet sind. Das Ergebnis ist insofern überraschend, als normalerweise die Transkription von Retroviren sowie das "capping" der genomischen RNAs im Zellkern stattfindet (Coffin et al., *supra*). Im gezeigten Retrovac-System finden Transkription und Capping durch Vaccinia-codierte Enzyme im Cytoplasma statt. Die *VV*-spezifischen Cap-Strukturen sind offensichtlich kein Hindernis für ein retrovirales Packaging.

### Beispiel 3:

### Konstruktion des VV-Defektvirus vd-e10A1

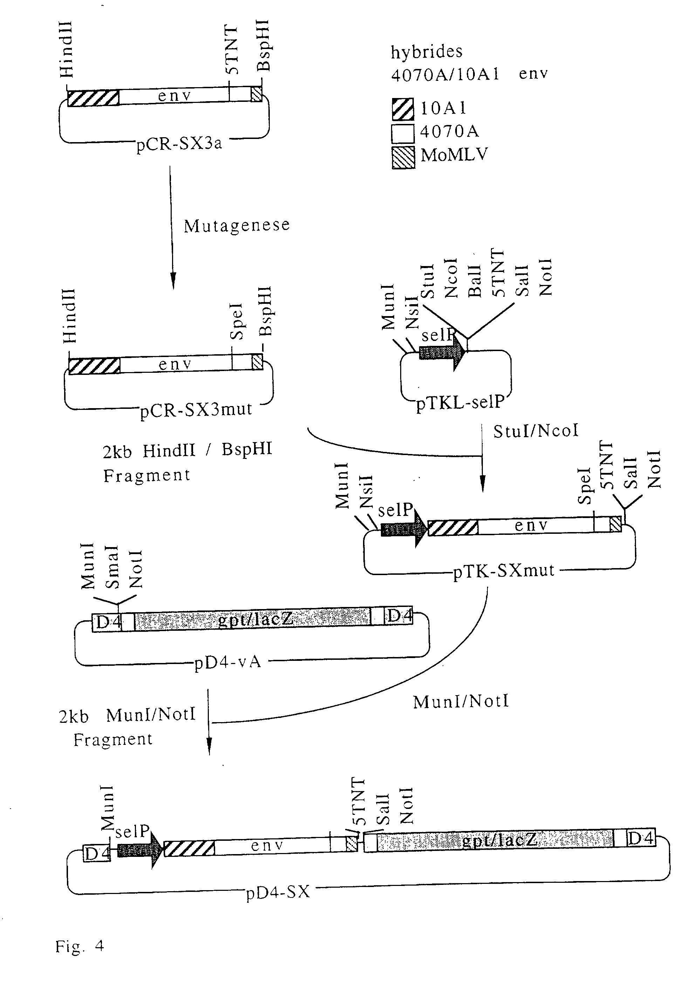

In den Beispielen 1 und 2 wurde gezeigt, daß defekte *VV*, die retrovirale Genome transkribieren, in Packaging-Zellinien funktionelle Defekt-Retroviren bilden. Im folgenden wurde das 10A1 env-Gen im D4-Locus von *VV* exprimiert, ein weiterer Schritt, alle Komponenten zur Bildung defekter RV-Partikel auf einem Defekt-*VV* zu vereinigen. Die Klonierung erfolgte in Zwischenschritten, da ein TTTTTNT-Motiv der env-Region deletiert werden mußte (Fig. 4).

### Konstruktion des Plasmids pD4-SX

Plasmid pCR-SX3mut: Das MLV env-Gen (env SX: Hybrid aus den Stämmen 4070A und 10A1) wurde als 2kb PCR-Produkt mit den Primern oRV-37 (5'-TCAGGGTCGAC ATGGAAGGTC CAGCGTTCTC-3') und oRV-38 (5'-GACTTTCATG ACTATGGCTC GTACTCTATA GGCTTC-3') aus der Zellinie PT67 (Miller et al., 1996, J. Viol. 70:5564-5571) amplifiziert und in den Plasmidvektor pCRII kloniert. Ein TTTTTNT-Signal im env-Leseraster wurde an diesem Plasmid durch Mutagenese abgewandelt. Die Mutagenese erfolgte mit dem QuickChange™-Mutagenesekit der Fa. Stratagene und den Primern oRV-41 (5'-GAATGTTGTT TCTATGCAGA CCACACGGGA CTAGTGAGAG-3') und oRV-42 (5'-CTCTCACTAG TCCCGTGTGG TCTGCATAGA AACAACATTC-3'). Das resultierende Plasmid wurde pCR-SX3mut genannt.

Plasmid pTK-SXmut: Aus Plasmid pCR-SX3mut wurde der env-Leseraster wurde als 2kb-Fragment mit den Enzymen BspHI und HindII ausgeschnitten und in das NcoI und StuI geschnittene Plasmid pTKL-selP ligiert und so Plasmid pTK-SXmut erhalten.

Plasmid pTKL-selP: Im Plasmid pTKgpt-selP (Beispiel 1) wurde ein 0,3kb-Fragment über die Schnittstellen HindIII und NdeI durch einen synthetischen Linker mit den Oligonucleotiden oRV-22 (5'-AGCTCGCAAT TGATGCATCA CA-3') und oRV-23 (5'-TATGTGATGC ATCAATTGCG-3') ersetzt und die zusätzlichen Schnittstellen NsiI und MunI einführt.

Plasmid pD4-SX: Eine Expressionskassette, bestehend aus dem starken *VV*-Promotor selP und dem env-Leseraster wurde mit den Enzymen MunI und NotI aus dem Plasmid pTK-SXmut ausgeschnitten und in MunI/NotI geschnittenes pD4-vA (21) ligiert. Der resultierende Vektor pD4-SX (Fig. 4) enthält somit die beschriebene Expressionskassette zwischen flankierenden Sequenzen des *VV*-D4-Locus. Integration dieser DNA in *VV* ergibt damit defekte, nicht replizierende Rekombinanten, die das env-Gen exprimieren.

### Konstruktion des VV-Defektvirus vd-e10A1

Die homologe Rekombination von pD4-SX mit dem *VV*-Wildtyp Western Reserve (WR) Stamm erfolgte nach Standardtechniken in RK-D4R-44.20-Zellen. Plaquereinigung erfolgte in RK-D4R-44.20-Zellen zweimal mit gpt-Selektion und Screening auf lacZ-positive Plaques und zweimal ohne Selektion und Screening auf lacZ-negative Plaques (Holzer et al., 1997, J. Virol. 71:4997-5002). Die resultierenden Isolate wurden mit vd-e10A1 bezeichnet. Kontrolle der env-Expression erfolgte im Westernblot. Ein MoMLV-spezifisches Antiserum wurde von Quality Biotech / Camden, NJ erhalten (Serum ID: 81S000044 "disrupted Virus").

### Beispiel 4:

### Konstruktion des VV-Defektvirus vd-eg

Der MLV gag-pol-Leseraster wurde in den Hämagglutinin-Genlocus des *VV*-Vektors vd-e10A1 (Beispiel 3) inseriert. Dadurch entsteht ein defektes *VV*, welches sowohl gag-pol^{MLV} als auch env^{10A1} exprimiert und Pseudopartikel bilden kann. Dieser MLV-Packagingvektor weist noch einen freien Thymidin-Kinase-Genlocus auf, in den retroviralen Defektgenome mit zur Gentherapie geeignete Fremdgene inseriert werden können (wie in Beispiele 5 und 6 beschrieben).

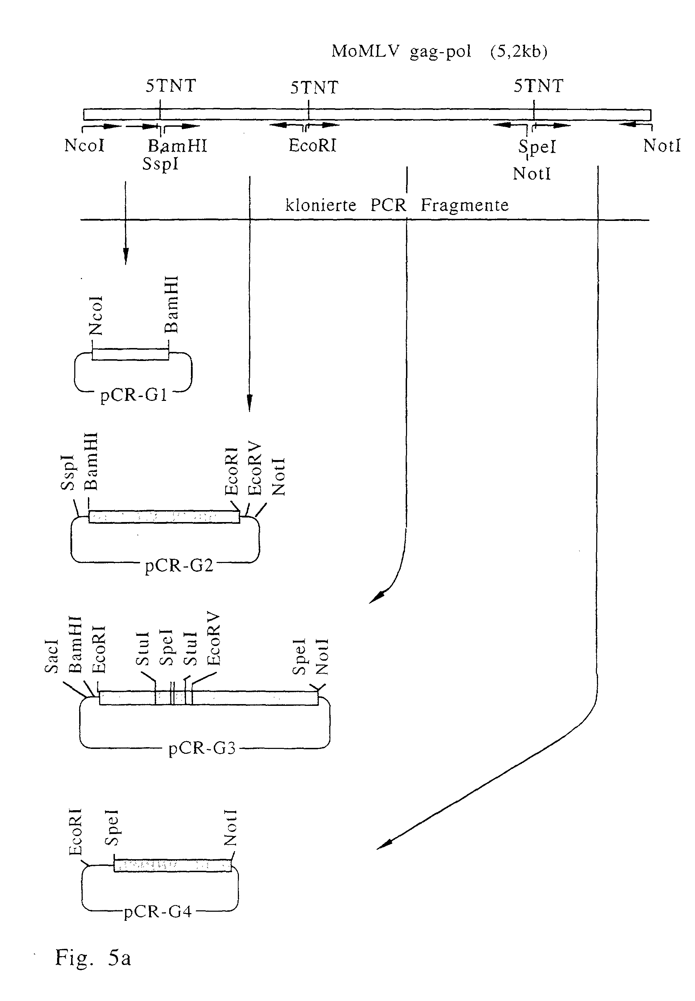

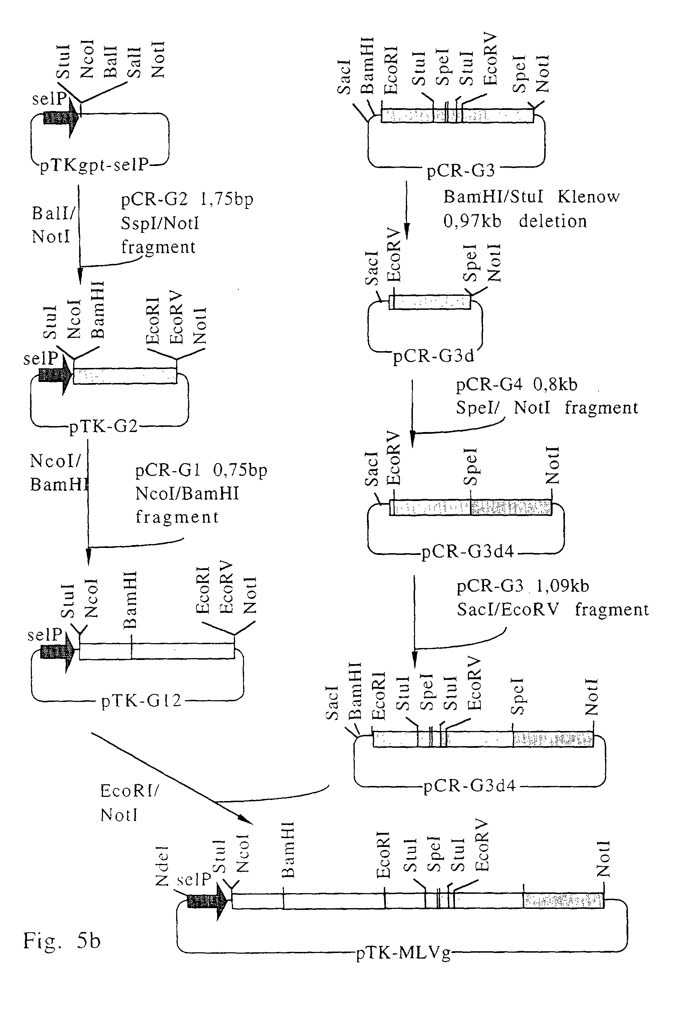

Die Klonierung erfolgte in Zwischenschritten, da drei TTTTTNT-Motive der gag-pol-Region deletiert werden mußten (Fig. 5a und 5b).

Das MLV gag-pol-Leseraster (5,2kb) wurde über PCR aus der packaging-Zellinie PT67 (Clontech Laboratories, Inc., Palo Alto, CA) amplifiziert. Dazu wurden vier PCR-Fragmente (G1-G4) amplifiziert, die an ihren Enden überlappen. Durch die Primer wurden die drei vorhandenen TTTTTNT-Motive abgewandelt und kompatible Schnittstellen generiert, ohne die Aminosäuresequenz zu beeinflussen.

Amplifikation des Fragments G1: Mit den Oligonucleotidprimern oRV-11 (5'-CCATGGGCCA GACTGTTACC ACT-3') und oRV-12 (5'-CTGGATCCTC AGAGAAAGAA GGGTT-3') wurde ein 0,8kb-Fragment aus MLV gag-pol generiert, wobei durch oRV-11 eine NcoI-Schnittstelle und durch oRV-12 ein TTTTTNT-Signal mutiert und eine BamHI-Schnittstelle eingeführt wurde.

Amplifikation des Fragments G2: Mit den Oligonucleotiden oRV-13 (5'-ATTAACCCTT CTTTTTCTGA GGATCCAGGT-3') und oRV-14 (5'-CATCCTTGAA TTCAAGCACA GTGTACCACT G-3') wurde aus MLV-gag-pol ein 1,7kb-Fragment generiert, wobei durch das Oligonukleotid oRV-13 eine BamHI-Schnittstelle eingeführt wird, sowie eine zusätzliche SspI-Schnittstelle, die es an seinem 3'-Ende aufweist; durch oRV-14 wird eine EcoRI-Schnittstelle eingeführt.

Amplifikation des Fragments G3: Mit den Oligonucleotidprimern oRV-15 (5'-TGAATTCAAG GATGCCTTCT TCTGCCTGA-3')und oRV-16 (5'-AACTAGTAGA TATTTATAGC CATAC-3') wurde aus MLV-gag-pol ein 2kb-Fragment generiert, wobei oRV-15 ein TTTTTNT-Signal mutiert und eine EcoRI-Schnittstelle einführt; durch oRV-16 wird eine SpeI-Schnittstelle und eine zusätzliche NotI-Schnittstelle, die es an seinem 3'-Ende aufweist, eingeführt.

Amplifikation des Fragments G4: Mit den Oligonucleotiden oRV-17 (5'-ATATCTACTA GTTTTCATAG ATACCT-3') und oRV-18 (5'-GCGGCCGCTT AGGGGGCCTC GCGGGTTA-3') wurde aus MLV gag-pol ein 0,8kb-Fragment generiert, wobei durch oRV-17 ein TTTTTNT-Motiv abgewandelt und eine SpeI-Schnittstelle und durch oRV-18 eine NotI-Schnittstelle eingeführt wurde.

### Klonierung der Plasmide pTK-MLVg und pHA-MLVg

Das G2 PCR-Fragment wurde in den Vektor pCRII (Invitrogen, Inc.) kloniert (pCR-G2) und daraus wieder über die Enzyme SspI und NotI isoliert (Fig. 5a). Ligation in den BalI und NotI geschnittenen Vektor pTKgpt-selP ergab das Plasmid pTK-G2 (Fig. 5b). Das G1 PCR-Produkt wurde mit den Enzymen NcoI und BamHI aus pCR-G2 ausgeschnitten, isoliert und in das mit NcoI und BamHI linearisierte Plasmid pTK-G2 ligiert, wodurch das Plasmid pTK-G12 erhalten wurde (Fig. 5b).

Das G3 PCR-Produkt wurde in den Vektor pCRII ligiert, um pCR-G3 zu erhalten. Durch Verdau mit den Enzymen BamHI und StuI, Klenow-Behandlung und Religation wurde aus pCR-G3 eine lkb-Sequenz deletiert und in das so erhaltene Plasmid pCR-G3d wurde über die Schnittstellen SpeI und NotI das 0,8kb große G4 PCR-Produkt inseriert. Das resultierende Plasmid wurde als pCR-G3d4 bezeichnet. Insertion eines 1,1kb-Fragmentes, das durch Verdau von pCR-G3 mit den Enzymen SacI und EcoRV erhalten wurde, in das SacI und EcoRV geschnittene Plasmid pCR-G3d4 ergab das Plasmid pCR-G34, das die 3'-Hälfte des MLV gag-pol-Leserasters enthält. Ein 3kb-Fragment wurde über die EcoRI- und NotI-Schnittstellen verdaut und Religation von pCR-G34 in das Plasmid pTK-12 umkloniert. Im resultierenden Plasmid pTK-MLVg ist das gesamte MLV gag-pol-Leseraster stromabwärts des starken "early-late" -*VV*-Promotors selP enthalten (Fig. 5b).

Das Plasmid pTK-MLVg wurde NdeI (partial) und NotI geschnitten und die selP-gag-pol-Kassette als 5,3kb-Fragment isoliert. Das Fragment wurde Klenow-behandelt und in den SnaBI-linearisierten *VV*-Integrations-Plasmidvektor pHA-vA ligiert. Das resultierende Plasmid wurde pHA-MLVg genannt.

### Konstruktion und Funktionalitätstest des Packagingvektors vd-eg.

Die homologe Rekombination der Plasmid-DNA pHA-MLVg und dem Ausgangsvirus vd-e10A1 (Beispiel 3), sowie die Plaquereinigung erfolgte nach Standardprotokoll in RK-D4R-44.20-Zellen (Holzer et al., 1997, J. Virol. 71:4997-5002.). Die gag-pol-Expression wurde im Westernblot bestätigt. Die gag-pol- und env-exprimierenden Isolate wurden mit vd-eg bezeichnet.

Weiters wurde die Funktionalität der exprimierten retroviralen Gene an 3T3-Zellklonen getestet, die durch Transformation mit dem retroviralen Vektor pLXSN erhalten worden waren. Dazu wurden 3T3-Zellen mit defektem LXSN-Retrovirus, wie in RetroXpress System User Manual. Clontech Laboratories, Inc., Palo Alto, CA(1997) beschrieben, transformiert. Fünf G418-resistente Klone wurden isoliert. Die Überstände dieser Kulturen waren nicht infektiös, konnten also keine Transduktion nativer 3T3-Zellen bewirken. Diese Linien wurden mit rekombinanten Viren vd-eg-infiziert (moi=1). Nach drei Tagen wurden die Überstände geerntet und zur Transformation von 3T3-Zellen eingesetzt. Die Transformationsexperimente erfolgten wie bei Miller et al. (1996. J. Virol. 70: 5564-5571) beschrieben. Die so transformierten 3T3-Zellen waren G418-resistent, die Überstände von selektierten Klonen waren nicht transformierend. Die beobachtete Transformation wurde also nicht durch eine Reaktivierung von vermehrungsfähigen Retroviren bewirkt. Dieses Experiment beweist, daß Expression der retroviralen Gene durch den Packagingvektor vd-eg das Verpacken transformierender retroviraler Defektgenome ermöglicht.

### Beispiel 5:

### Konstruktion des Retrovac-Vektors vd-egLXSN

Mit vd-egLXSN als Ausgangsvektor wurde ein *VV*-Vektor konstruiert, der alle notwendigen Komponenten für Bildung von transduzierenden Defekt-retroviralen Partikel exprimiert. Die Herstellung von vd-egLXSN erfolgte über homologe Rekombination mit der Plasmid-DNA pTKgpt-LXSN (Beispiel 1) und dem Packagingvirus vd-eg (Beispiel 4) nach Standardprotokoll in RK-D4R-44.20-Zellen (siehe Beispiel 1). Isolate wurden in mehreren Plaquereinigungsrunden in RK-D4R-44.20-Zellen unter gpt-Selektion gereinigt und mit vd-egLXSN bezeichnet. Mit Saccharosegradient-gereinigten Virus-Stocks von vd-egLXSN wurden 3T3- und RK13-Zellen infiziert (moi=1). Nach 6h wurden die Überstände geerntet und analog zu Beispiel 1 auf Transformation von 3T3-Zellen getestet. Die Überstände von 10⁶ -Zellen enthielten je nach Zellinie zwischen 10² und 10³ CFU-retroviralen Vektor. Dieses Beispiel beweist, daß die Infektion von Wildtyp-Zellen mit dem Retrovac-Vektor vd-egLXSN ausreicht, um transformierende retrovirale Vektorpartikel zu exprimieren.

### Beispiel 6:

### Konstruktion eines Retrovac-Vektors vd-egC9, der dRV-Partikel mit Faktor IX cDNA-Insert exprimiert.

Im folgenden Experiment wird der humane Gerinnungsfaktor FIX in einen Retrovac-Vektor kloniert und retrovirale Defektpartikel erzeugt, die zur Gentherapie von Hämophilie B geeignet sind. Dazu wird eine Genkassette, bestehend aus dem Cytomegalovirus (CMV) Promotor/Enhancer (Boshart et al., 1985. Cell. 41:521-530) und der Gerinnungsfaktor FIX-cDNA und genetischen Elementen ("die FIX-Genkassette", wie in der EP 0 711 835 beschrieben), welche die RNA-Prozessierung erlauben und in das Plasmid pTKgpt-VP inseriert. Das resultierende Plasmid pTKgpt-VP-FIX wird in den tk-Locus des Packagingvektors vd-eg rekombiniert, resultierend im Retrovac-Vektor vd-egC9.

### Konstruktion des Plasmids pTKgpt-VP-FIX

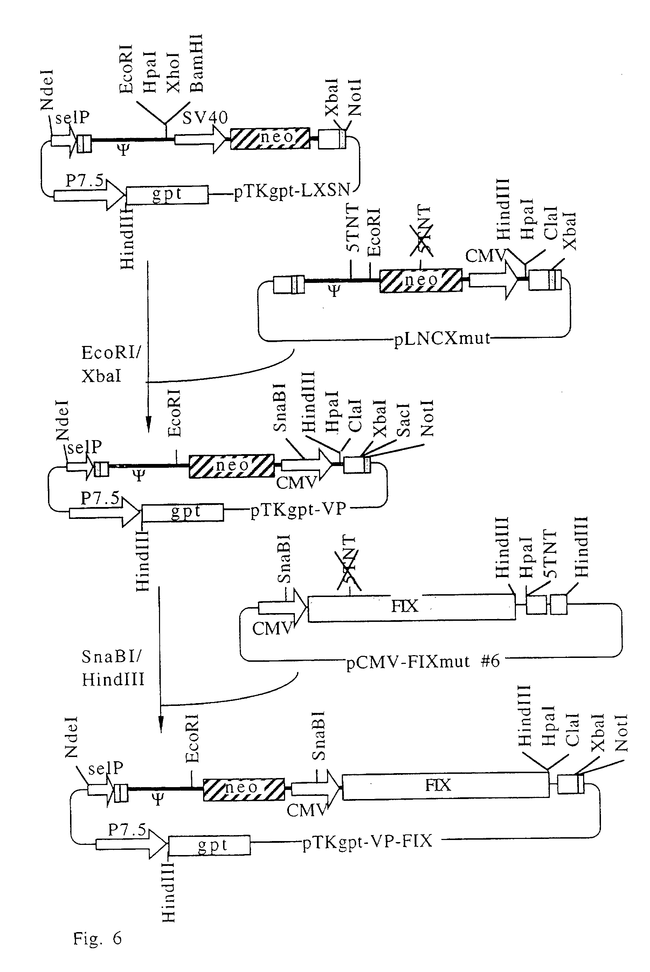

Die Faktor IX-cDNA (Kurachi et al., 1982, Proc. Natl. Acad. Sci., USA, 79:6461-6464) enthält ein TTTTTNT-Motiv, das über Mutagenese mit den Primern omut.FIX-1 und omut.FIX-2 abgewandelt wurde (Fig. 6). Das resultierende Plasmid wurde mit pCMV-FIXmut bezeichnet.

pTKgpt-VP. Analog zu pTKgpt-LXSN wurde ein Plasmidvektor für VV-Expression auf der Basis von pLNCX konstruiert. Ein TTTTTNT-Motiv im Neomycin-Resistenz-Gen von pLNCX wurde zu diesem Zweck mutagenisiert (primer oRV-35 und oRV-9). Die rechte Hälfte der retroviralen Vektorsequenz wurde aus diesem mutagenisierten Plasmid pLNCXmut über die Schnittstellen EcoRI und XbaI in den Vektor pTKgpt-LXSN umgesetzt. Das resultierende Plasmid wurde pTKgpt-VP genannt und enthält die Elemente *VV*-Thymidin-Kinase (tk) Genflanke / *VV*-Promotor selP / RV-'R' / RV-U5 / Psi / Neo /CMV Promotor / MCS / RV U3 / RV-'R' / TTTTTNT / *VV*-tk-Flanke.

Zur Konstruktion von Plasmid pTKgpt-VP-FIX wurde ein SnaBI-/HindIII-Fragment, das einen Teil des CMV-Promotors und die gesamte FIX-cDNA enthält, aus pCMV-FIXmut in SnaBI / HindIII (Partialverdau) geschnittenes pTKgpt-VP gesetzt.

### Konstruktion und Test des Retrovac-Vektors vd-egC9.

Das Plasmid pTKgpt-VP-FIX wurde nach Standardmethoden in den Packagingvektor vd-eg rekombiniert und Plaque gereinigt (siehe Beispiel 5). Das resultierende Virus wurde vd-egC9 bezeichnet. Sucrosegradient-gereinigte Stocks von vd-egC9 wurden präpariert um 3T3- und RK13-Zellen zu infizieren (moi=1). Nach 3 Tagen wurden die Überstände geerntet und analog zu Beispiel 4 auf Transformation von 3T3-Zellen getestet. Die Überstände von 10⁶⁻Zellen enthielten je nach Linie zwischen 10² und 10³ CFU-retroviralen Vektor. Infektion von halbkonfluenten RK13 (ATCC CCL-37)- oder SK-HEP-1 (ATCC HTB-52)-Zellen mit den Überständen, die retrovirale FIX-Defektpartikel enthielten, exprimierten funktionellen Faktor IX (gemessen wie in der EP 0 711 385 beschrieben).

### Beispiel 7:

### Infusion des Retrovac-Vektors vd-egC9 in Kaninchen und Nachweis von humanem Faktor IX im Kaninchenplasma.

Im folgenden Experiment wird im Tierversuch gezeigt, daß der Retrovac-Vektor vd-egC9 in vivo die Expression von Faktor IX erlauben.

New Zealand white-Kaninchen wurden 10⁸ pfu bzw. 10⁹ pfu vd-egC9 intravenös verabreicht und alle zwei Tage wurde über einen Zeitraum von einem Monat Blut abgenommen und hu Faktor IX im ELISA quantifiziert. Prä-Infusionsseren wurden genommen, um festzustellen, ob der Elisa spezifisch ist für den humanen Faktor IX. Humaner Faktor IX konnte im Kaninchenplasma in Konzentrationen festgestellt werden, die für eine Hämophilie B-Therapie ausreichend sind. Die Präsenz von anti-human-Plasmaprotein-Äntikörpern wurde nach vier Wochen in Westernblots festgestellt. Im Kontrollexperiment (Infektion mit dem Defektvirus vD4-vA) konnte kein humaner Faktor IX nachgewiesen werden.

## Patentansprüche

1. Chimäres Poxvirus, **dadurch gekennzeichnet, dass** es alle Sequenzen eines defekten retroviralen Genoms, die für die Expression des retroviralen Genoms notwendig sind, enthält, wobei eine Fremdsequenz unter der transkriptionellen Kontrolle eines Zellkern-aktiven Promotors in das retrovirale Genom inseriert ist.

2. Chimäres Poxvirus nach Anspruch 1, **dadurch gekennzeichnet, dass** es die Sequenz der Verpackungskomponenten gag, pol und env eines Retrovirus enthält.

3. Chimäres Poxvirus nach einem der Ansprüche 1 oder 2, **dadurch gekennzeichnet, dass** das Poxvirus ein Chordopoxvirus, vorzugsweise ein Orthopoxvirus, ist.

4. Chimäres Poxvirus nach Anspruch 3, **dadurch gekennzeichnet, dass** das Orthopoxvirus ein Vaccinia-Virus ist.

5. Chimäres Poxvirus nach einem der Ansprüche 1 bis 4, **dadurch gekennzeichnet, dass** es ein defektes Vaccinia-Virus ist.

6. Chimäres Poxvirus nach einem der Ansprüche 1 bis 5, **dadurch gekennzeichnet, dass** die Sequenz des retroviralen Genoms unter transkriptioneller Kontrolle eines Poxvirus-Promotors steht.

7. Chimäres Poxvirus nach einem der Ansprüche 3 bis 5, **dadurch gekennzeichnet, dass** die Sequenz der Verpackungskomponenten unter transkriptioneller Kontrolle eines Poxvirus-Promotors steht.

8. Chimäres Poxvirus nach einem der Ansprüche 6 oder 7, **dadurch gekennzeichnet, dass** der Poxvirus-Promotor ein früher Poxvirus-Promotor ist.

9. Chimäres Poxvirus nach einem der Ansprüche 1 bis 8, **dadurch gekennzeichnet, dass** das retrovirale Genom Intronsequenzen enthält.

10. Chimäres Poxvirus nach einem der Ansprüche 1 bis 8, **dadurch gekennzeichnet, dass** das retrovirale Genom eine interne Polyadenylierungssequenz enthält.

11. Zusammensetzung enthaltend ein chimäres Poxvirus, das alle Sequenzen eines defekten retroviralen Genoms, die für die Expression des retroviralen Genoms notwendig sind, sowie die Sequenz der Verpackungskomponenten gag, pol und env eines Retrovirus enthält, wobei eine Fremdsequenz unter der transkriptionellen Kontrolle eines Zellkern-aktiven Promotors in das retrovirale Genom inseriert ist, und einen pharmazeutisch akzeptablen Träger.

12. Verwendung eines chimären Poxvirus nach einem der Ansprüche 1 bis 10 zur Herstellung eines Arzneimittels.

13. Verwendung eines chimären Poxvirus nach einem der Ansprüche 1 bis 10 zur Herstellung eines Arzneimittels zum Einsatz in der Gentherapie.

14. Verwendung eines chimären Poxvirus nach einem der Ansprüche 1 bis 10 zur Herstellung eines Arzneimittels zum Einsatz in der Tumortherapie.

## Claims

1. A chimeric poxvirus, **characterized in that** it comprises all the sequences of a defective retroviral genome which are required for the expression of the retroviral genome, wherein a foreign sequence is inserted into the retroviral genome under the transcriptional control of a nucleus-active promotor.

2. A chimeric poxvirus according to claim 1, **characterized in that** it comprises the sequence of the packaging components gag, pol and env of a retrovirus.

3. A chimeric poxvirus according to any one of claims 1 or 2, **characterized in that** the poxvirus is a chordopox virus, preferably an orthopox virus.

4. A chimeric poxvirus according to claim 3, **characterized in that** the orthopox virus is a vaccinia virus.

5. A chimeric poxvirus according to any one of claims 1 to 4, **characterized in that** it is a defective vaccinia virus.

6. A chimeric poxvirus according to any one of claims 1 to 5, **characterized in that** the sequence of the retroviral genome is under the transcriptional control of a poxvirus promoter.

7. A chimeric poxvirus according to any one of claims 3 to 5, **characterized in that** the sequence of the packaging components is under the transcriptional control of a poxvirus promoter.

8. A chimeric poxvirus according to any one of claims 6 or 7, **characterized in that** the poxvirus promoter is an early poxvirus promoter.

9. A chimeric poxvirus according to any one of claims 1 to 8, **characterized in that** the retroviral genome comprises intron sequences.

10. A chimeric poxvirus according to any one of claims 1 to 8, **characterized in that** the retroviral genome comprises an internal polyadenylating sequence.

11. A composition comprising a chimeric poxvirus which comprises all the sequences of a defective retroviral genome which are required for the expression of the retroviral genome, as well as the sequence of the packaging components gag, pol and env of a retrovirus, wherein a foreign sequence is inserted into the retroviral genome under the transcriptional control of a nucleus-active promotor, and a pharmaceutically acceptable carrier.

12. The use of a chimeric poxvirus according to any one of claims 1 to 10 for preparing a medicament.

13. The use of a chimeric poxvirus according to any one of claims 1 to 10 for preparing a medicament to be applied in gene therapy.

14. The use of a chimeric poxvirus according to any one of claims 1 to 10 for preparing a medicament to be applied in tumor therapy.

## Revendications

1. Poxvirus chimérique **caractérisé en ce qu'**il contient l'ensemble des séquences d'un génome rétroviral défaillant lesquelles séquences sont nécessaires pour l'expression du génome rétroviral, dans lequel une séquence étrangère est insérée sous le contrôle transcriptionnel d'un promoteur actif du noyau cellulaire dans le génome rétroviral.

2. Poxvirus chimérique **caractérisé en ce que** l'orthopoxvirus chimérique selon la revendication 1, **caractérisé en ce qu'**il contient la séquence des composants de conditionnement gag, pol, et env d'un rétrovirus.

3. Poxvirus chimérique selon l'une quelconque des revendications 1 à 2, **caractérisé en ce que** le poxvirus est un chordopoxvirus de préférence un orthopoxvirus.

4. Poxvirus chimérique selon la revendication 3, **caractérisé en ce que** l'orthopoxvirus est un virus de la vaccine.

5. Poxvirus chimérique selon l'une quelconque des revendications 1 à 4, **caractérisé en ce qu'**il s'agit d'un virus de la vaccine présentant des défaillances.

6. Poxvirus chimérique selon l'une quelconque des revendications 1 à 5, **caractérisé en ce que** la séquence du génome rétroviral se trouve sous le contrôle transcriptionnel d'un promoteur de poxvirus.

7. Poxvirus chimérique selon l'une quelconque des revendications 3 à 5, **caractérisé en ce que** la séquence des composants de conditionnement se trouve sous le contrôle transcriptionnel d'un promoteur poxvirus.

8. Poxvirus chimérique selon l'une quelconque des revendications 6 à 7, **caractérisé en ce que** le promoteur poxvirus est un promoteur poxvirus précoce.

9. Poxvirus chimérique selon l'une quelconque des revendications 1 à 8, **caractérisé en ce que** le génome rétroviral contient des séquences d'introns.

10. Poxvirus chimérique selon l'une des revendications 1 à 8, **caractérisé en ce que** le génome rétroviral contient une séquence de polyadénylisation.

11. Composition contenant un poxvirus chimérique qui présente l'ensemble des séquences d'un génome rétroviral présentant des défaillances lesquelles séquences sont requises pour l'expression du génome rétroviral, ainsi que la séquence des composants de conditionnement gag, pol et env d'un rétrovirus dans laquelle composition une séquence étrangère est insérée sous contrôle transcriptionnel d'un promoteur actif du noyau cellulaire au sein du génome rétroviral, et une substance vectrice acceptable d'un point de vue pharmaceutique.

12. Utilisation d'un poxvirus chimérique selon l'une quelconque des revendications de 1 à 10 pour la fabrication d'un médicament.

13. Utilisation d'un poxvirus chimérique selon l'une quelconque des revendications 1 à 10 pour la fabrication d'un médicament destiné à la thérapie génétique.

14. Utilisation d'un poxvirus chimérique selon l'une des revendications de 1 à 10 pour la fabrication d'un médicament destiné à un traitement anticancéreux.
